# EUROPEAN PATENT APPLICATION

(11) **EP 1 097 941 A1**
(43) Date of publication of application: **09.05.2001**
(21) Application number: 99122141.7
(22) Date of filing: 05.11.1999
(51) Int. Cl.: C07K 14/47, C12N 15/12, C12N 9/12, C12N 15/54, C12N 5/10, C12Q 1/68

(54) **Substance capable of controlling the inclusion of exon 10 of the tau gene, and its therapeutic use against tauopathies**

(71) Applicant: Max-Planck-Gesellschaft zur Förderung der Wissenschaften e.V., 80539 München (DE)
(72) Inventor: Stamm, Stefan Dr., 81375 München (DE); Andreadis, Athena, Dr., Cambridge, MA 02140 (US); Goedert, Michel, Dr., Cambridge CB4 1EU (GB); Hartmann, Annette, 80809 München (DE); Stoilov, Peter, 82152 Planegg (DE); Stoss, Oliver, 81377 München (DE)
(74) Representative: Oser, Andreas

(57) **Abstract**

The present inventions relates to substances which are capable of controlling the inclusion of exon 10 of the tau gene. The substances can reverse "wrong" tau splicing pattern and. Since "wrong" tau splicing patterns are associated with tauopathies that cause dementia, the substances can be used as therapeutic agents. The invention also provides a method for testing substances that control tau exon 10 inclusion. The method is suitable for a high throughput screening. Additional products which are useful for changing tau exon 10 expression or for performing the test method are described as well.

SEQUENCE LISTING

<110> Max-Planck-Gesellschaft z. Förd. d. Wissenschaften

<120> Substances for the Treatment of Tauopaties

<130> EP25609

<140>
<141>

<160> 20

<170> PatentIn Ver. 2.1

<210> 1
<211> 113
<212> DNA
<213> Artificial Sequence

<220>
<223> Description of Artificial Sequence: tau exon 10
      and 3' proximal intron junction

<400> 1
gtgcagataa ttaataagaa gctggatctt agcaagctcc agtccaagtg tggctcaaag 60
gataatatca aacacgtccc gggaggcggc agtgtgagta ccttcacacg tcc 113

<210> 2
<211> 20
<212> DNA
<213> Artificial Sequence

<220>
<223> Description of Artificial Sequence:
      Oligonucleotide

<400> 2
aggtactcac actgccgcct 20

<210> 3
<211> 22
<212> DNA
<213> Artificial Sequence

<220>
<223> Description of Artificial Sequence:
      Oligonucleotide

<400> 3
aagatccagc ttcttattaa tt                                              22

<210> 4
<211> 22
<212> DNA
<213> Artificial Sequence

<220>
<223> Description of Artificial Sequence:
      Oligonucleotide

<400> 4
gactatttgc accttcccgc ct                                              22

<210> 5
<211> 25
<212> DNA
<213> Artificial Sequence

<220>
<223> Description of Artificial Sequence:
      Oligonucleotide

<400> 5
ggcttgagaa cagcccaggg agttc                                           25

<210> 6
<211> 25
<212> DNA
<213> Artificial Sequence

<220>
<223> Description of Artificial Sequence:
      Oligonucleotide

<400> 6
gaattcgagc aagtagcggg tccag                                           25

<210> 7
<211> 24
<212> DNA
<213> Artificial Sequence

<220>
<223> Description of Artificial Sequence:
      Oligonucleotide

<400> 7
gaattctatg cggtgtctcg caag                                              24

<210> 8
<211> 24
<212> DNA
<213> Artificial Sequence

<220>
<223> Description of Artificial Sequence:
      Oligonucleotide

<400> 8
agtctcaggt gccaccacct tcag                                              24

<210> 9
<211> 26
<212> DNA
<213> Artificial Sequence

<220>
<223> Description of Artificial Sequence:
      Oligonucleotide

<400> 9
gtctgggtct tgcacaatga caatgg                                            26

<210> 10
<211> 24
<212> DNA
<213> Artificial Sequence

<220>
<223> Description of Artificial Sequence:
      Oligonucleotide

<400> 10
atgttgccta atgagccaca cttg                                              24

<210> 11
<211> 26
<212> DNA
<213> Artificial Sequence

<220>
<223> Description of Artificial Sequence:
      Oligonucleotide

<400> 11
gcagataatt aagaagaagc tggatc                                          26

<210> 12
<211> 27
<212> DNA
<213> Artificial Sequence

<220>
<223> Description of Artificial Sequence:
      Oligonucleotide

<400> 12
gaagctggat ctgagcaacg tccagtc                                         27

<210> 13
<211> 27
<212> DNA
<213> Artificial Sequence

<220>
<223> Description of Artificial Sequence:
      Oligonucleotide

<400> 13
ccgggaggcg gcaatgtgag taccttc                                         27

<210> 14
<211> 35
<212> DNA
<213> Artificial Sequence

<220>
<223> Description of Artificial Sequence:
      Oligonucleotide

<400> 14
ggcagtgtga gtaccttcat acgtcccatg cgccg                                35

<210> 15
<211> 35
<212> DNA
<213> Artificial Sequence

<220>
<223> Description of Artificial Sequence:
      Oligonucleotide

<400> 15
ggcagtgtga gtaccttcac atgtcccatg cgccg                                35

<210> 16
<211> 35
<212> DNA
<213> Artificial Sequence

<220>
<223> Description of Artificial Sequence:
      Oligonucleotide

<400> 16
ggcaatgtga gtaccttcac atgtcccatg cgccg                                35

<210> 17
<211> 24
<212> DNA
<213> Artificial Sequence

<220>
<223> Description of Artificial Sequence:
      Oligonucleotide

<400> 17
gggagcccaa gaaggtggca gtgg                                            24

<210> 18
<211> 30
<212> DNA
<213> Artificial Sequence

<220>
<223> Description of Artificial Sequence:
      Oligonucleotide

<400> 18
tagcgtggcg agctggggcg agtctaccat                                      30

<210> 19
<211> 30
<212> DNA
<213> Artificial Sequence

<220>
<223> Description of Artificial Sequence:
      Oligonucleotide

<400> 19
cagctacagt cggaaaccat cagcaagcag                                        30

<210> 20
<211> 30
<212> DNA
<213> Artificial Sequence

<220>
<223> Description of Artificial Sequence:
      Oligonucleotide

<400> 20
cacctccagt gccaaggtct gaaggtcacc                                        30

## Description

Tau is a microtubule-associated protein (MAP) enriched in axons of mature and growing neurons (Binder et al., J. Cell Biol. 101, 1371-8 (1986); Kempf et al., J. Neurosci. 16, 5583-92 (1996)). Tau has also been found in the cell nucleus (Wang et al., J. Cell Biol. 121, 257-67 (1993)), in the distal ends of growing neurons (Black et al., J. Neurosci. 16, 3601-19 (1996); DiTella et al., Cell Motil. Cytoskel. 29, 117-30 (1994)), in oligodendrocytes (LoPresti et al., Proc. Natl. Acad. Sci. USA 92, 10369-73 (1995)) and in muscle (Wei & Andreadis, 1998). Hyperphosphorylated, microtubule-dissociated tau protein is the major component of neuro-fibrillary tangles, a hallmark of several tauopathies (neurodegenerative diseases; reviewed in Spillantini & Goedert, Trends Neurosci. 21, 428-33 (1998)).

Tau is encoded by a single copy gene (Himmler, Mol. Cell. Biol. 9, 1389-96 (1989); Neve et al., J. Mol. Brain Res. 1, 271-80 (1986)). The structure of the human tau gene is disclosed by Andreadis, A. et al. in Biochemistry 31, 10626-33 (1992). Tau transcripts of 2, 6 and 9 kb are differentially expressed in the nervous system, depending upon stage of neuronal maturation and neuron type (Couchie et al., Proc. Natl. Acad. Sci. USA 89, 4378-81 (1992); Drubin et al., J. Cell Biol. 106, 1583-91 (1988); Goedert et al., Neuron 3, 519-26 (1989), Goedert et al., EMBO J. 8, 393-99 (1989), Goedert et al., Neuron 21, 955-8 (1998); Himmler et al., Mol. Cell Biol. 9, 1389-96 (1989); Wang et al., *supra).* The 2 kb tau mRNA is localized to the nucleus (Wang et al., *supra).* The 9 kb tau transcript is restricted to the retina and peripheral nervous system (reviewed in Nuñez and Fischer, J. Mol. Neurosci. 8, 207-22 (1997).

The tau transcripts undergo complex, regulated alternative splicing: six of the sixteen tau exons are regulated cassette exons and the gene utilizes two alternative polyadenylation sites which correspond to the 2 and 6 kb tau mRNA (Andreadis et al., *supra* and Andreadis et al., Nucl. Acids Res. 23, 3585-93 (1995); Couchie et al., *supra;* Goedert et al., *supra;* Himmler, *supra;* Himmler et al., *supra;* Kosik et al., Neuron 2, 1389-97 (1989); Lee et al., Science 239, 285-8 (1988); Sadot et al., J. Mol. Biol. 241, 325-31 (1994)).

The N-terminus of the tau protein is acidic and interacts with the plasma membrane (Brandt et al., J. Cell Biol. 131, 1327-40 (1995)), although the specific molecules with which it interacts are not yet known. The C-terminus of the tau protein is basic and contains four imperfect repeats (encoded by exons 9 to 12) each of which acts as microtubule binding domains (Himmler et al., *supra;* Lee et al., Neuron 2, 1615-24 (1989)).

Exon 10 is a cassette which codes for an additional microtubule binding domain. Its inclusion increases the affinity of tau for microtubules and the stability of the tau-micro-tubule interaction (Lee et al., *supra).* Recent findings have established that missplicing of tau exon 10 can cause inherited frontotemporal dementia with parkinsonism (FTDP-17), almost certainly by disturbing the normal tau isoform ratio (Clark et al., Proc. Natl. Acad. Sci. USA 95, 13103-7 (1998); Hasegawa et al., FEBS Lett. 443, 93-6 (1999); Hutton et al., Nature 393, 702-5 (1998); Spillantini et al., *supra).* The FTDP pedigree mutations initially clustered at or near the 5' splice site of exon 10, giving rise to the hypothesis that its splicing is partly modulated by a putative hairpin loop, whose formation inhibits interaction with the U1 snRNP (Hutton et al., *supra;* Grover et al., J. Biol. Chem. 274, 15134-43 (1999). However, others have shown that exon 10 splicing is also affected by an exonic sequence that is identical to a silencer motif found in the HIV *tat* gene (D'Souza et al., Proc. Natl. Acad. Sci. USA 96, 5598-603 (1999)).

The discovery that sulfated glucosaminoglycans and RNA induce bulk assembly of full-length recombinant tau into Alzheimerlike filaments has provided a first experimental system for assembly of tau into filaments (reported in Spilantini & Goedert, *supra).*
Based on this discovery, an assay for the testing of compounds that prevent tau filament formulation was proposed. However, this biochemical assay is not suitable for an efficient screening method in order to test a high number of substances which may be useful as a therapeutic agent for treating tauopathies.

It is an object of the present invention to provide a substance which is suitable as a therapeutic agent for the treatment of tauopathies, such as frontotemporal dementia, Parkinsonism, Alzheimer disease or Pick's disease. A further object of the present invention to provide a process which is suitable for modifying the tau isoform pattern of a mammalian cell.

Another object of the present invention is to provide a method which is suitable for a high throughput screen for substances that can be effective for the treatment of tauopathies, along with products which are suitable for such a screening method.

The present invention provides a substance which is capable of controlling the inclusion of exon 10 of the tau gene for use as a therapeutic agent.

The present invention further provides a process for changing the pre-mRNA processing relating to the tau gene of a mammalian cell, which comprises exposing the cell with the afore-mentioned substance.

The present invention also provides a mammalian host cell which is transfected with a DNA being selected from the group of DNAs comprising (a) DNAs encoding polypeptides which are capable of at least partially suppressing the inclusion of exon 10 of the tau gene, and (b) DNAs encoding polypeptides which are capable of controlling the phosphorylation of a splicing regulator for exon 10 of the tau gene. The transfected mammalian host cell, which preferably originates from a human cell culture and/or from a human individual to be treated (especially from human brain cells), is useful in gene therapy.

The present invention also makes available a useful, high throughput screening method for the testing of substances that control the inclusion of exon 10 of the tau gene, along with useful products being particularly suitable for such a screening method.

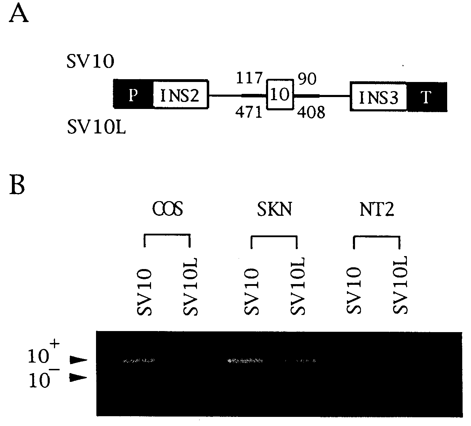

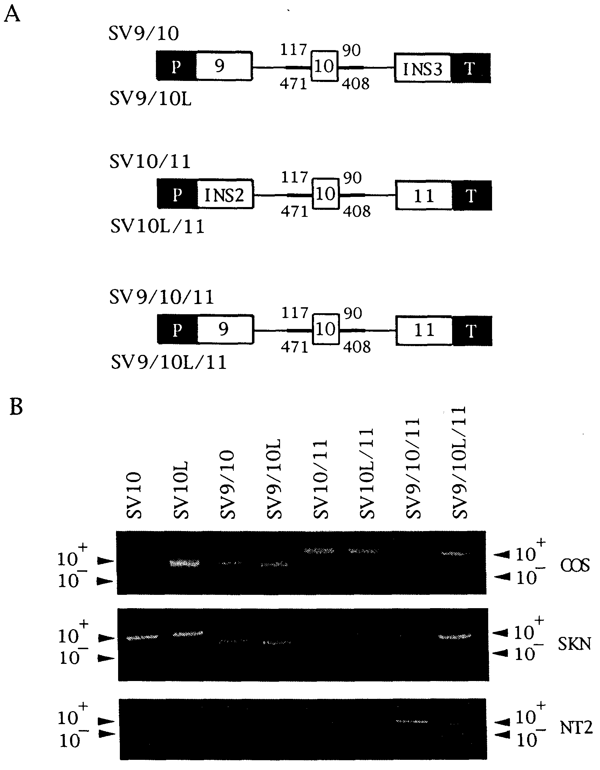

The present invention will now be described in further detail while referring to the accompanying figures, where
Fig. 1A and 2A show schematic representations of various, tau exon 10 containing minigene expression constructs, while Fig. 1B and 2B shows the corresponding tau isoform ratios between exon 10 inclusion and exon 10 exclusion obtained from these constructs in different mammalian cells.

3A shows the tau exon 10 sequence (in capital letters) with a part of its adjacent downstream intron sequence (in small letters), along with mutant forms thereof, which sequences have been used for minigene constructions, while Fig. 3B the corresponding tau isoform ratios between exon 10 inclusion and exon 10 exclusion obtained from these constructs, and Figs. 4 and 5 show the effects of specific substances according to the present invention on the control of tau exon 10 inclusion.

According to the present invention, it was surprisingly found that the "wrong" splicing pattern associated with exon 10 of the human tau gene, i.e. the imbalance between the inclusion or exclusion of tau exon 10 for the final tau protein, can be reverted by certain substances which are capable of controlling the inclusion of exon 10 of the tau gene. Accordingly, the substance according to the present invention enables the re-instatement of the balance between tau isoforms having four microtubule binding repeats (i.e. inclusion of exon 10) and tau isoforms with three microtubule binding repeats (i.e. exclusion of exon 10). Since isoforms with four microtubule binding repeats have a higher affinity for microtubules and, therefore, uncontrolled overproduction of tau with four microtubule binding repeats may lead to disease states of tauopathies such as frontotemporal dementia, Parkinsonism and Alzheimer's disease, down-regulation of exon 10 inclusion (i.e. exon 10 skipping) generates a tau isoform pattern that is less prone to filament formation and subsequent neuronal degeneration. While exon 10 skipping provides a basis for treating the afore-mentioned pathological states being characterized by an overproduction of tau with four microtubule binding repeats, the opposite approach of at least partially stimulating the inclusion of exon 10 of the tau gene can counter-act a sub-representation of tau with four microtubule repeats which is typical for other neurodegenerative disease states such as Pick's disease.

On account of the individual to be treated, the tau gene and, correspondingly, its exon 10 to be controlled should be of human origin, as only humans show alternative splicing of tau exon 10. The total human tau gene can be derived from Genebank accession No. X14474. Parts of the total tau gene are disclosed in Genebank accession Nos. AF047855-AF047863 and AF027491-AF027496. The sequence of tau exon 10 is shown under Genebank accession No. AF027494.

The substance according to the present invention may directly or indirectly control *in vivo* the inclusion of exon 10 of the tau gene. In the direct control, the substance may directly affect pre-mRNA processing or may directly interact with any one of the splicesome components involved in tau exon 10 splicing. Alternatively, since tau exon 10 splicing is regulated *in vivo* in a complex regulative system comprising specific kinase and phosphatase enzymes, the. controlled generation, or the inhibition or the stimulation of these specific kinase and/or phosphatase enzymes are appropriate indirect control means for tau exon 10 splicing.
Included within the meaning of such an indirect control is the expression of polypeptide factors through genetic engineering from their respective encoding polynucleotides (usually in the form of DNA, especially cDNA or genomic DNA), which polypeptide factors in turn interact with exon 10 splicing either directly or indirectly, as will be described below. Furthermore, "controlling" the inclusion of exon 10 of the tau gene according to the present invention means either an at least partial suppression, or an at least partial stimulation of inclusion of tau gene exon 10. In other words, the alternative splicing pattern of tau is changed either in favor or against exon 10 skipping, respectively. Accordingly, the "wrong" splicing pattern and, therefore, the imbalance between tau isoforms having four or three microtubule binding repeats can be reverted, thus rendering the substances of the present invention particularly useful as therapeutic agents.

Embodiments for the substance which meet the above-mentioned features and objects will now be described in further detail.

The substance according to the present invention may be a splicing regulator for the splicing of exon 10 of the tau gene. Table 1 below shows a list of splicing regulators that may affect tau exon 10 splicing (only the name of the gene is given, while it is apparent that most genes have associated splice variants).

**Table 1**

| Factor | Source |
|---|---|
| SPp20 | Ayane, M. et al., Nucl. Acids Res. *19*, 1273-1278 (1991) |
| | |
| | |
| ASF/SF2 | Krainer, A.R. et al., Genes and Dev. *4,* 1158-1171 (1990) |
| | |
| SC35 | Fu, X.D. and Maniatis, T. Science *256,* 535-538 (1992) |
| | |
| SRp30c | Screaton, G.R. et al., EMBO J. *14,* 4336-4349 (1995) |
| | |
| SRp40 | Screaton, G.R. et al., EMBO J. *14,* 4336-4349 (1995) |
| | |
| SRp55 | Screaton, G.R. et al. EMBO J. *14,* 4336-4349 (1995) |
| | |
| SRp75 | Zahler, A.M. et al. Mol. Cell. Biol. *13,* 4023-4028 (1993) |
| | |
| 9G8 | Cavaloc, Y. et al. EMBO J. *13,* 2639-49 (1994) |
| | |
| tra2-beta | Beil, B. et al., DNA and Cell Biol. *16,* 679-690 (1997) |
| | |
| tra2-alpha | Dauwalder, B. et al., Proc. Natl. Acad. Sci. USA *93,* 9004-9009 (1996) |
| | |
| SWAP | Sarkissian, M. et al., J. Biol. Chem. *271,* 31106-31114 (1996) |
| | |
| TASR | Yang, L. et al., J. Biol. Chem. 27761-27764 (1998) |
| | |
| PTB | Valcarcel, J. and Gebauer, F. Current Biol. *7,* R705-708 (1997) |
| | |
| SF-1 | Wang, X. et al., EMBO J. *18,* 4549-4559 (1999) |
| | |
| SLM-1 | Di Fruscio, M. et al. Proc. Natl. Acad. Sci. USA *96,* 2710-2715 (1999) |
| | |
| SLM-2 | Di Fruscio, M. et al., Proc. Natl. Acad. Sci. USA *96,* 2710-2715 (1999) |
| | |
| YT521-B | Hartmann, A.M. et al., Mol. Biol. Cell in press (1999) |
| | |
| U170K | Spritz, R.A. et al., Genomics *8,* 371-379 (1990) |
| | |
| U2AF65 | Zamore, P.D. et al., Nature *355,* 609-614 (1992) |
| | |
| U2AF35 | Zhang, M. et al., Proc. Natl. Acad. Sci. *89,* 8769-8773 (1992) |

According to a preferred embodiment of the present invention, the substance is capable of at least partially suppressing the inclusion of the exon 10 of the tau gene. This leads to exon 10 skipping and, therefore, to a down-regulation of tau protein isoforms having four microtubule binding repeats. We have found regulators which specifically inhibit exon 10 splicing and, accordingly, promote exon 10 skipping. Specifically, among the factors listed in Table 1 above, the following splicing regulators suppress the inclusion of tau exon 10: The serine/arginine-rich proteins (SR proteins) ASF/SF2, SRp55 and SRp75; U2AF65; and hSWAP. Among the polypeptides suitable for exon 10 skipping are also the fragments, derivatives or variants of the afore-mentioned polypeptides which are effective for suppressing the inclusion of exon 10 of the tau gene.

Alternatively, the substance according to the present invention is represented by a polynucleotide (DNA, especially cDNA or genomic DNA) which respectively encodes for the splicing regulator mentioned above, preferably a DNA which encodes for the inhibitory splicing regulator for tau exon 10, particularly a DNA which respectively encode for any one of U2AF65, hSWAP and the SR proteins ASF/SF2, SRP55 and SRP75. Of course, DNAs are included which are defined by fragments, derivatives, alleles or mutant forms of the afore-mentioned DNAs, which fragments, derivatives, alleles or mutant forms are effective for suppressing the inclusion of tau exon 10.

According to another embodiment of the present invention, the substance is a polypeptide which controls the phosphorylation of a splicing regulator for the splicing of tau exon 10, or a DNA encoding such a polypeptide (including fragments, derivatives or mutant forms thereof being effective for controlling the phosphorylation degree of a tau exon 10 splicing regulator). A preferred example for such a phosphorylation controlling substance (thereby indirectly controlling exon 10 inclusion) is a SR protein kinase. It is assumed that phosphorylation of the SR protein-type splicing regulators results in a recruitment of those factors from intracellular storage pools, known as speckles. Phosphorylation may be controlled directly by means of kinases which phosphorylate the splicing regulators, such as SR protein kinases. It was surprisingly found that cd2-like SR protein kinases (types clkl-4, for example clk2), which are known per se (see Nayler 0. et al. (1997), Biochem. J. 326, 693-700), lead to an at least partial suppression of inclusion of tau exon 10 by being particularly effective for exon 10 skipping. Conversely, instead of these kinase enzymes, phosphatases which specifically dephosphorylate a tau exon 10 splicing regulator are suitable for stimulating the inclusion of tau exon 10. In this connection, the substance for controlling the phosphorylation of a splicing regulator, and thereby controlling tau exon 10 inclusion, is preferably a SR protein phosphatase, or a DNA encoding therefor (including fragments, derivatives or mutant forms thereof being effective for controlling the phosphorylation degree of a tau exon 10 splicing regulator). While the cd2-like kinases (clkl-4) lead to the phosphorylation of the SR protein splicing factors, the corresponding phosphatases result in a de-phosphorylation of the clk targets. By means of clk-mediated phosphorylation or de-phosphorylation, the active concentration and/or phosphorylation degree of the SR protein targets can be changed and adjusted *in vivo,* thereby resulting in an indirectly controlled change of exon 10 inclusion or exclusion of the tau gene.

Further information on SR and SR-like proteins is provided in articles of Fu, X.D., RNA 1, 663-680 (1995) and Kramer A., Annu. Rev. Biochem. 65, 367-409 (1996).
Other molecules which interact with clk2 and which may, therefore, be suitable as regulating substances for the present invention are the proteins corresponding to the genes dan26 (Genbank Accession No. U94836) and son-a (Genbank Accession No. X63071), and DNAs encoding therefor.

Since several such substances have been isolated for other systems, low molecular weight substances putatively exist which either stimulate the afore-mentioned kinase enzymes such as the cd2-like SR protein kinases, or block the corresponding phosphatase. Accordingly, particularly suitable substances for the purpose of the present invention are inhibitors of the kinase and phosphatase enzymes mentioned above. The phosphorylation degree of tau exon 10-specific splicing regulators, e.g. the afore-mentioned SR proteins, is in turn affected by these kinase and phosphatase inhibitors, consequently resulting again in the controlled change of exon 10 inclusion of the tau gene. This has been established in the present invention by changing the clk2 phosphorylation levels by means of okadaic acid and 5,6-dichloro-1-b-D-ribofuranosylbenzimidazole (DRB). The low molecular weight substances can be applied as therapeutic agents suitably in appropriate pharmaceutical formulations.

According to another embodiment of controlling tau exon 10 inclusion or exon 10 skipping, oligonucleotides which interact with the splice site of tau exon 10 can be applied and are therapeutically useful. More specifically, antisense oligonucleotides and oligonucleotides for triplex formation or aptamers are suitable for this embodiment, as described basically by Schmajuk G. et al., J. Biol. Chem. 274, 21783-21789 (1999) and Kole R., Acta Biochem. Pol. 44, 231-237 (1997). The target to be treated is preferably exposed to an antisense oligonucleotide being complementary to the splice site which is defined by the junction between the 3'-end of tau exon 10 and the 5'-end of the flanking downstream intron sequence. The oligonucleotide preferably has a length of 15 to 25 nucleotides, e.g. a 20-mer oligonucleotide, while it spans the aforementioned splice site region in the form of a complementary sequence. For example, an appropriate therapeutic oligonucleotide sequence is the following (SEQ ID No. 2): 5' AGGTACTCACACTGCCGCCT 3'.
In order to improve stability of the oligonucleotide in vivo, the oligonucleotide preferably has a morpholino backbone (see Schmajuk G. et al., *supra).*

The present invention also provides a process for controlling tau exon 10 inclusion by changing the pre-mRNA processing relating to the tau gene of a mammalian cell, which process comprises exposing the cell to one of the substances specified above. The mammalian cell is preferably a human cell, particularly a human brain cell. When the DNAs described above are used as the substance, the mammalian cell is transfected in an appropriate transformation/transfection system known to those skilled in the art. The mammalian cells may be transiently transfected, but the respective DNA is preferably stably integrated in the cells. For this purpose, the mammalian cell may be exposed with the substance outside the body to be treated (ex *vivo).* The above described DNA substances of the present invention may also be used in vectors which have been developed for gene therapy such as the vectors for delivering therapeutic genes described by Mitani et al. in TIBTECH 11, 162-166 (1993). In such a vector, the DNA is usually operatively linked to an expression control DNA sequence known to those skilled in the art.

Accordingly, the present invention also provides a mammalian host cell which is stably transfected with a DNA, especially with a vector containing such DNA, where the DNA is as specified above.

The present invention also advantageously provides a method for the testing of substances that control the inclusion of exon 10 of the tau gene, which method comprises the steps of (1) transfecting mammalian cells with a DNA which comprises exon 10 of the tau gene or fragments or mutant forms thereof, (2) exposing the transfected cells obtained from step (1) to the substance to be tested, and (3) determining the ratio between exon 10 inclusion and exon 10 skipping as a result of exposure step (2). This method has been successfully applied for verifying the effectiveness of substances specified above for modifying tau exon 10 splicing, and it can also be advantageously used for finding further substances which may directly or indirectly control the inclusion of tau exon 10. The test method according to the invention is particularly designed to be suitable for a high throughput screening for substances which putatively control tau exon 10 inclusion or skipping. The transfected cells may be incubated in plates having a high number of wells and treated with substances from a chemical library, especially substances having a relatively low molecular weight.

The transfection of mammalian cells, which are preferably human cells and more preferably cells derived from the human brain, may be carried out by a conventional method known to those skilled in the art.

The present invention also provides, in a preferred embodiment, a minigene construct which is particularly suitable for transfecting the mammalian cells to be used for the afore-mentioned screening method. While the DNA used for transfection requires the presence of the tau exon 10 sequence of the tau gene or fragments or mutant forms thereof, the preferred minigene construct comprises, as additional DNA elements, each intron sequence respectively flanking tau exon 10 upstream and downstream (or fragments or mutant forms of the respective intron sequences) as well as exon 9 and/or exon 11 of the tau gene (or fagments or mutant forms derived from exon 9 and/or exon 11). The minigene construct according to the present invention may further comprise at least one further foreign DNA element differing from the tau gene, which foreign DNA element(s) serve as an internal splicing control. As another DNA element which is suitable for an internal control minigene construct, an exon 10 mutant form may be used which constitutively leads to exon 10 inclusion.

Examples for minigene constructs according to the present invention are schematically shown in Figs. 1A and 2A and described in more detail in Example 1 below.

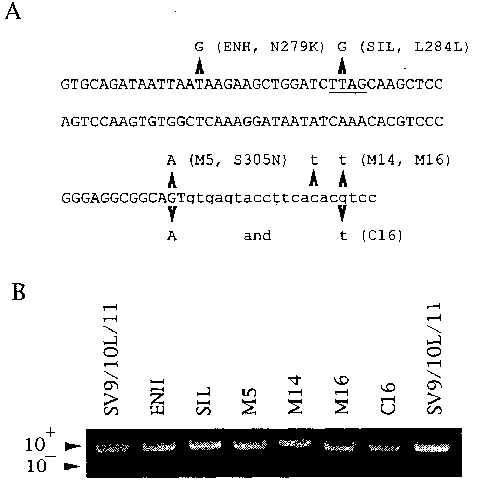

The tau exon 10 sequence with a part of its adjacent downstream intron sequence is given in SEQ ID No. 1, and exemplary mutant forms based on these sequences are shown in Figure 3A, i.e. mutations affecting exon 10 itself: ENH, the N279K mutation which creates a purine-rich enhancer; SIL, the L284L silent mutation which destroys a tat-like silencer (the underlined sequence is the motif homologous to the *tat* silencer); M5, the S305N mutation which creates a 5' splice site capable of much better binding to the Ul snRNP than the wild type; see Clark et al., *supra;* D'Souza et al., *supra;* Grover et al., *supra;* and Hasegawa et al., *supra;* as well as mutations within the proximal 3' intron: M14 and M16 affecting intron positions +14 and +16, respectively; see Hutton et al., *supra;* as well as "compensatory" commutation to the +16 mutant (C16), which would restore the structure of the putative hairpin-like structure forming around the 5' splice site of exon 10 (Grover et al., *supra).*

When fragments of the flanking intron sequences are to be inserted into the minigene construct, relatively long fragments comprising the flanking upstream and downstream intron sequences of tau exon 10, respectively, are preferred.

The tau minigene constructs may be produced by standard cloning methodology of by PCR cloning. Examples for suitable PCR primers to be used for wild-type and mutant constructs are described in Example 1 below.

The afore-mentioned DNA elements should be fused into a plasmid or a vector suitable for transfecting the desired mammalian cells.

It has been surprisingly found according to the present invention that the default splicing pattern of exon 10 is inclusion independently of the length of the flanking introns. Furthermore, it was found that both flanking exons 9 and 11 are involved in the regulation of exon 10 splicing. More specifically, inclusion of pre-fused exon 9 into the minigene construct resulted in the exclusive production of the tau isoforms containing exon 10. On the other hand, inclusion of pre-fused exon 11 resulted in a roughly 1:1 balanced ratio of exon 10 inclusion and exon 10 exclusion in neuroplastoma cells. Therefore, not only minigene constructs containing tau exon 10 and at least partial sequences of the flanking introns upstream and downstream of exon 10, respectively, but minigene constructs which additionally contain exon 9 and/or exon 11, or fragments or mutant forms of these DNA sequences, are particularly useful for the test method according to the present invention.

In the test method, after the transfected mammalian cells had been exposed to a substance which putatively changes tau exon 10 splicing and, therefore, putatively controls tau exon 10 inclusion (step (2) mentioned above), the ratio between exon 10 inclusion and exon 10 skipping is determined (step (3)). Accordingly, the suitability and the effectiveness of the substance is checked. The tau exon 10 inclusion/skipping ratio may be determined as follows.

First, RNA obtained from the transfected and exposed cell is subjected to reverse transcription (RT). Then, the DNA obtained from reverse transcription is amplified such that exon 10 sequences of the tau gene may be amplified, if present, and the thus amplified DNA is subjected to DNA analysis which is capable of distinguishing between exon 10 inclusion and exon 10 skipping of the tau gene. Reverse transcription can be carried out by conventional methods, and the DNA amplification is preferably carried out by means of PCR with suitable primer pairs, e.g. those specifically described below in Example 4.

After the DNA fragments relevant for exon 10 inclusion or skipping have been amplified, an appropriate DNA analysis is performed which allows to distinguish between exon 10 inclusion and exon 10 skipping of the tau gene.

If a DNA analysis on a qualitative laboratory scale is desired, conventional DNA electrophoresis may be conducted in order to separate and, thus, distinguish the differently sized, amplified fragments specific for either exon 10 inclusion or exon 10 skipping. Since RT-PCR primer pairs being specific for the respective exons adjacent to exon 10 are used (i.e. one primer specific for exon 9 and the other primer being specific for exon 11), the generated DNA amplicons may contain exon 10 sequences (thereby producing relatively large DNA amplicons due to exon 10 inclusion) or may lack exon 10 sequences (thereby producing relatively small DNA amplicons due to exon 10 skipping). The DNA amplicons differing in size can be separated by conventional gel electrophoresis, and the ratio between the large DNA amplicon and the relatively small DNA amplicon, corresponding to the ratio between exon 10 inclusion and exon 10 skipping, can be determined, e.g. by DNA band staining. More preferably, a semi-quantitative or quantitative analysis is applied by conducting DNA analysis by at least two oligonucletide probes, one probe being specific for exon inclusion and one probe being specific for exon skipping. The probe which is specific for exon inclusion spans the tau exon 10 sequence of appropriate length, e.g. 15 to 25 nucleotides long, and the probe being specific for exon skipping spans the exon 9/exon 11 junction of the tau gene, i.e. covers a part of the 3'-end region of exon 9 and a part of the 5'- end region of exon 11 to provide an oligonucleotide probe of appropriate length, e.g. 15 to 25 nucleotides long.

In a preferred embodiment of the determination step (3), molecular beacon probes are used to provide an efficient and quantitative DNA analysis which can be carried out automatically. Molecular beacons are single-stranded nucleic acid molecules that possess a stem-and-loop structure (as described by Tyagi, S. and Kramer, F.R. in Nature
Biotechnology 14, 303-308 (1996). The loop portion of the molecule serves as a probe sequence that is complementary to the specific target nucleic acid. The stem is formed by the annealing of two complementary arm sequences that are on either side of the probe sequence. A fluorescent moiety is attached to the end of one arm, and a fluorescence quenching moiety is attached to the other arm. The stem hybrid keeps the fluorescer and the quencher so close to each other that fluorescence does not occur. When the molecular beacon probe encounters the specific target molecule, it forms a probe-target hybrid that is stronger and more stable than the stem hybrid. The probe then undergoes a spontaneous conformational re-organization that forces the arm sequence apart, separating the fluorophore from the quencher, and permitting the fluorophore to fluoresce. Accordingly, the molecular beacons may hybridize only to the specific target sequences that are perfectly complementary to the probe sequence, and the PCR reaction products can be analyzed and determined in a homogeneous assay in hermetically sealed tubes, thereby avoiding contaminations. Using this method eliminates the need for gel electrophoresis and allows for automation. Using a pair of molecular beacon probes having differently colored fluorophors (see Tyagi, S. et al., Nature Biotechnology 16, 49-53 (1998)), the presence or absence of exon 10 in the amplicons obtained in step (3) of the present screening method can be determined simultaneously in one assay. That is, one molecular beacon probe is target-specific for the tau exon 10 sequence, and the other molecular beacon probe is target-specific for the tau exon 9/exon 11 junction. An optimized molecular beacon probe pair is the following, wherein the molecular beacon recognizing tau exon 10 inclusion is TAMRA-cagcgcAAGATCCAGCTTCTTATTAATTgcgctg-6-FAM, and the molecular beacon recognizing tau exon 10 skipping is

TAMRA-cgcgagcGACTATTTGCACCTTCCCGCCTgctcgcg-TET.

In these exemplified molecular beacon probes, the respective target-specific hybridizing sequence is shown in capital letters (SEQ ID Nos. 3 and 4), whereas the sequences shown in small letters correspond to the complementary stem part of the molecular beacon probe. Of course, the stem sequences can be replaced by appropriate alternative complementary stem portions. TAMRA (6-carboxy-tetramethylrodamine) denotes the moiety which quenches either one of the respective specific fluorophore moieties, 6-FAM (6-carboxy-fluorescein) and TET (tetrachloro-6-carboxy fluorescein). The fluorophore moieties which differ in their emitted fluorescence wavelength enables to distinguish between the two molecular beacon probes. Alternative to 6-FAM and TET, 2,7-dimethoxy-4,5-dichlor-6-carboxyfluorescein (JOE) and hexachloro-6-carboxyfluorescein (HEX) may be used as fluorophore moieties as well. As a further alternative, a target-specific hybridizing sequence being complementary to the above specified molecular beacon probes may be used as well.

Instead of the molecular beacon approach, another homogeneous PCR detection system called TaqMan (described in Livak K.J. et al. in "PCR Methods Applic." 4, 357-362 (1995)), which is also suitable for being conducted in hermetically sealed tubes, may be applied as well.

As a further product which is particularly suitable for the test method described above, the invention provides a mammalian host cell which is already transfected with the minigene construct specified above. The minigene construct, preferably containing tau sequences of human origin, especially from human brain tissue, may be transiently transfected, but is more preferably stably integrated into the mammalian cells.

Since the substances according to the present invention, which are capable of controlling the inclusion of exon 10 of the tau gene of a mammalian cell, especially human cell and in particular human brain cell, are suitable for reverting the tau-associated filamentous lesions, the following disease states can be contemplated for therapeutic treatment:
Alzheimer's disease, corticobasal degeneration, dementia pugilistica, dementia with tangles only, dementia with tangles and calcification, Down syndrome, frontotemporal dementias and Parkinsonism linked to chromosome 17, myotonic dystrophy, Niemann-Pick disease type C, Parkinsonism-dementia complex of Guam, Pick's disease, postencephalitic Parkinsonism, Prion diseases with tangles, progressive supranuclear palsy and subacute sclerosing panencephalitis.

Among these tauopathies to be treated, those substances which are capable of at least partially suppressing the inclusion of tau exon 10 are suitable for treating tauopathies which are characterized by an overproduction of tau isoforms with four binding repeats, especially frontotemporal dementia, Parkinsonism and/or Alzheimer's disease. On the other hand, those substances which are capable of at least partially stimulating the inclusion of tau exon 10 are particularly suitable for treating tauopathies which are characterized by a lack or under-representation of tau isoforms having four microtubule binding repeats, especially Pick's disease.

The present invention will now be described in further detail by way of the following, non-limiting examples.

### Example 1

### Plasmid and minigene construction and generation of mutant forms relating to tau exon 10

The human tau genomic fragments originated from cosmid or clones (Andreadis et al., *supra).* The parental vector used for cloning was pSVIRB (Andreadis et al., *Nucl. Acids Res.* 21, 2217-21 (1993); Andreadis et al. *Nucl. Acids Res.* 23, 3585-93. (1995); Wei & Andreadis, *supra).* pSVIRB contains an additional intron between fused SV40/insulin exon 1 and insulin exon 2 that is not shown in the figures, but which served as an internal splicing control.

Tau chimeric constructs were produced by standard cloning methodology or by PCR, insertion into pKS(+) Bluescribe (Stratagene) and subsequent cloning by directional ligation (Sambrook et al., *Molecular Cloning: A Laboratory Manual.* 2nd edition. Cold Spring Harbor Laboratory Press, Cold Spring Harbo, New York, 1989). The primers used for PCR are listed and shown in Table 2a. All inserts generated by PCR were sequenced, to ensure absence of mutations.

The following minigene constructs were generated and are diagramatically shown in Figures 1A and 2A. In the Figures P represents the SV40 early promoter, and T the insulin terminator. The small intron between the insulin 1 and 2 exons is not shown for the sake of clarity. The numbers on each side of tau exon 10 show how many nucleotides of its flanking introns are present. In Fig. 2A, the fusions between insulin 2 and exon 9 or tau exon 11 and insulin 3 are not shown for the sake of clarity. Conventions for the constructs are as in Fig. 1A.

SV10 contains tau exon 10 and the proximal portions of its flanking introns (117 bp upstream and 90 bp downstream) inserted into SVIRB. SV9/10 has the 3' 161 nucleotides of tau exon 9 and ∼1.5 kbp of its downstream intron fused to insulin exon 2. SV10/11 has the 5' 68 nucleotides of tau exon 11 and 324 nucleotides of its upstream intron fused to insulin exon 3. SV9/10/11 contains both the exon 9 and 11 fusions. All three are identical to SV10 with respect to the exon 10 insert.

The SV10L series constructs (SV10L, SV9/10L, SV10L/11, SV9/1OL/11) are identical to their SV10 homologues, except that they contain longer portions of the introns flanking exon 10 (471 bp upstream and 408 bp downstream).

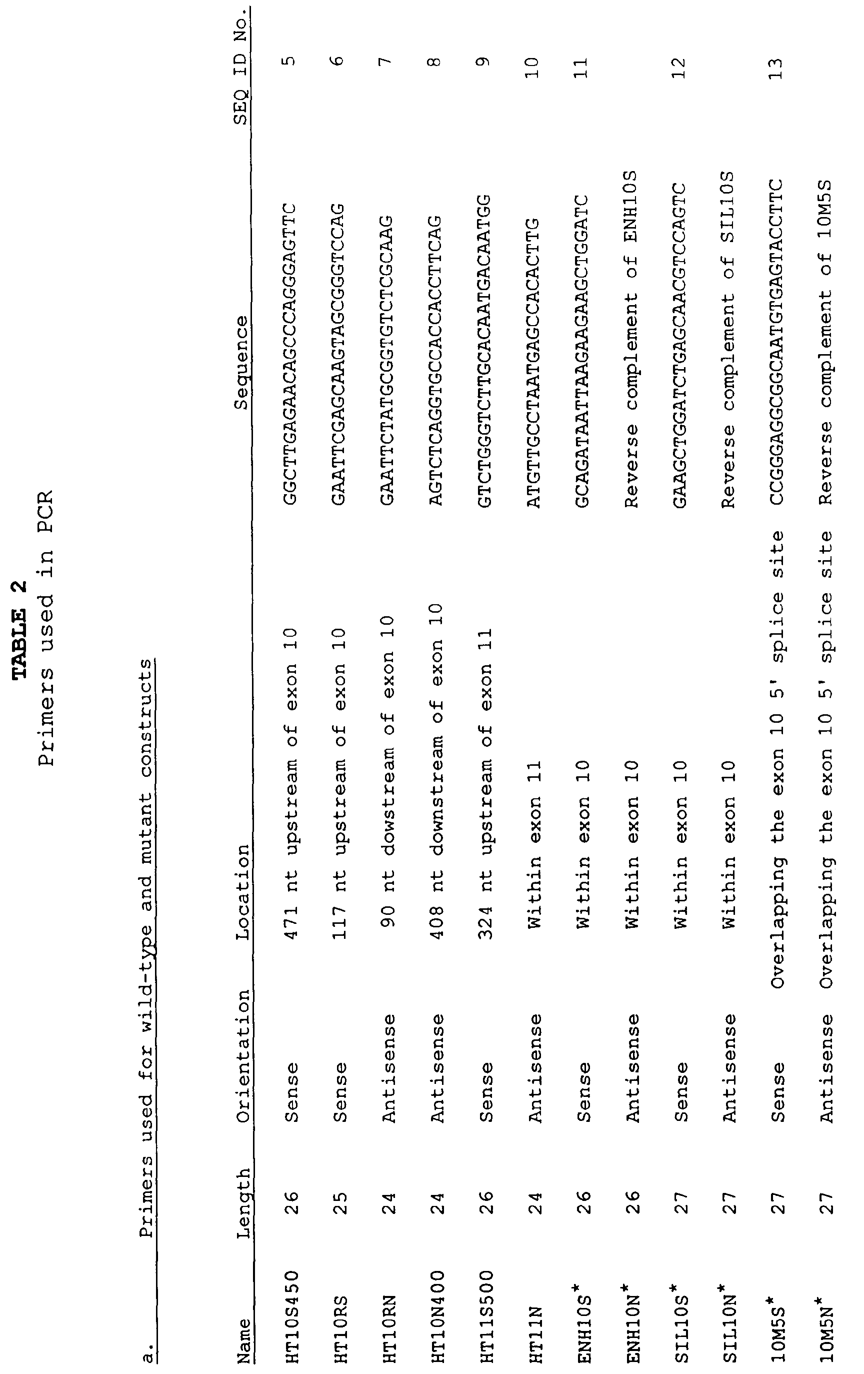

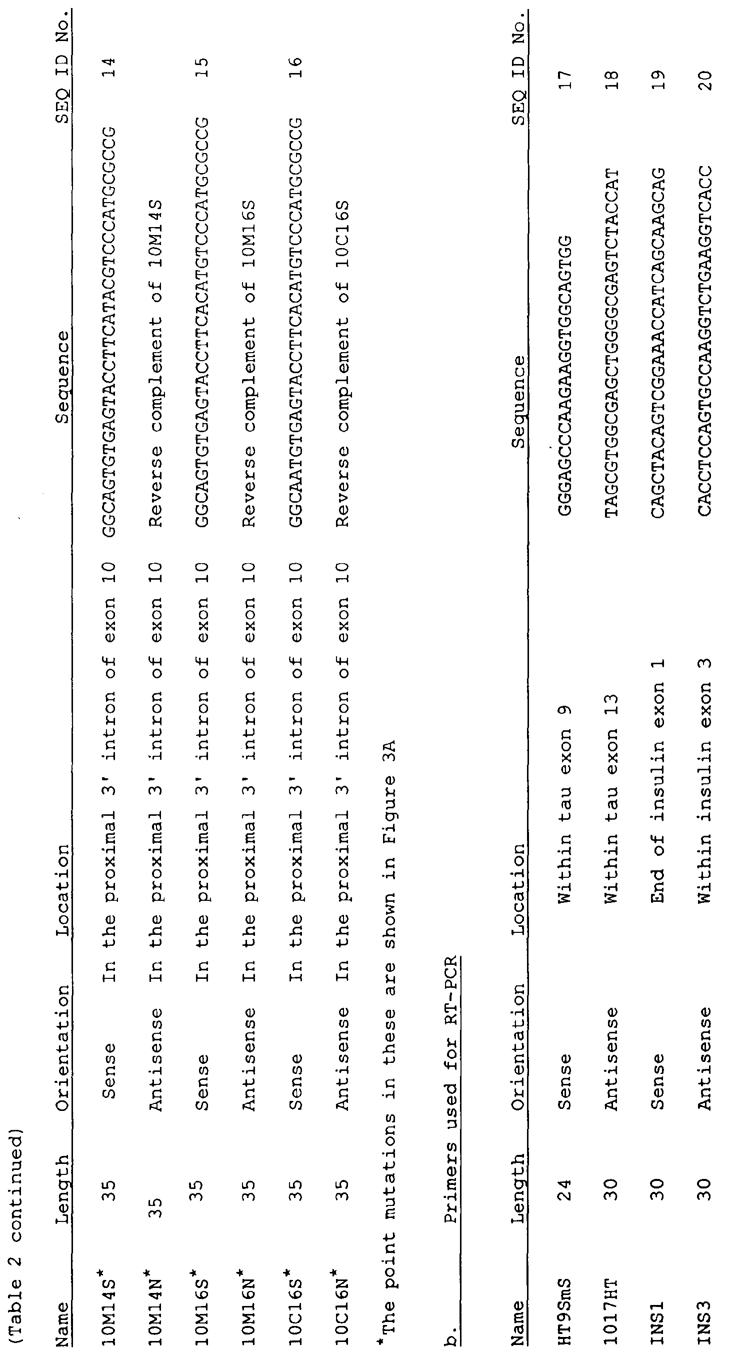

To generate the mutant constructs, whole-plasmid mutagenesis was performed on SV10 or SV9/10L/11 using the QuikChange Site Directed Mutagenesis kit (Stratagene) according to the manufacturer's instructions, except that the PCR products were digested with DpnI overnight. The resulting plasmids were sequenced to verify the presence of the desired mutations and the absence of undesirable ones. The primers used for mutageneses are listed in Table 2a and the alterations to wild-type exon 10 (cf. SEQ ID No. 1) are shown in Figure 3A.

### Example 2

### cDNA and vector constructions of tau exon 10 splicing regulators

The cDNA of putative splicing regulator Nova (Buckanovich & Darnell, 1997) was isolated from an adult human brain cDNA 5'-RACE-ready library (Clontech), placed into vector pCRII (InVitrogen), sequenced and subsequently placed into expression vector pCI-neo under the control of the CMV promoter. Production of the Nova protein was confirmed by the coupled transcription-translation reticulocyte system (Promega). Eukaryotic expression vectors bearing other splicing regulator cDNAs expressed them from the following promoters: CMV (for expression of hnRNPA1, U2AF65, htra2; SR proteins 20, ASF/SF2, 30c, SC35, 40, 55, 75; Caceres et al., 1997; Nayler et al., 1998; Screaton et al., 1995; Yang et al., 1994), adenovirus major late (for expression of Polypyrimidine Tract Binding Protein [PTB]; Patton et al., 1991) and SV40 early (for expression of Suppressor of White Apricot [SWAP]; Lemaire et al., 1999).

### Example 3

### Transfections of mammalian cells

COS (monkey kidney) and N-Tera2 cells (human teratocarcinoma, henceforth NT2) were maintained in Dulbecco's modified Eagle medium supplemented with 10% FCS. SK-N-SH cells (human neuroblastoma, henceforth SKN) were maintained in Eagle's minimal essential medium supplemented with non-essential amino acids and 10% FCS.

Plasmid DNA was purified by cesium chloride banding (Sambrook et al., 1989). The plasmids obtained in Examples 1 and 2 were introduced into cells by the lipofection method (LT1, Panvera).

Plates (100 mm) that had reached ∼30 % confluence were transfected with 10 mg of construct DNA or 5 mg of each plasmid for co-transfections. The medium was changed 16 h after transfection, without glycerol shock.

### Example 4

### Tau pre-mRNA processing analysis subsequent to transfection with tau exon 10 containing minigene constructs

Total RNA was isolated 48 h post-transfection by the TRIzol method (Life Technologies).

Reverse transcription and PCR reactions were performed with 1) human polyA⁺ RNA (Clontech) to establish the expression profile of endogenous tau exons and 2) total RNA from transfected cells to examine the behavior of the splicing constructs.

For PCR analysis of RNA, 5 mg of total RNA from transiently transfected cells or 1 mg of polyA⁺ RNA were reverse transcribed using random hexamer primers and 200 units of reverse transcriptase (RNAase H⁻ Superscript, Life Technologies), in a total volume of 20 ml for 1 h at 42 °C. Part of this reaction mix (5 ml) was then diluted to a final volume of 50 ml, the concentrations of the buffer and dNTPs were adjusted for PCR and the mixture was amplified for 22 cycles. The conditions were: for the endogenous gene, denaturation at 94 °C for 1 min, annealing at 58 °C for 1 min, extension at 72 °C for 1 min; for the constructs, denaturation at 94 °C for 1 min, annealing at 65 °C for 1 min, extension at 72 °C for 1 min.

The primer pair used for the endogenous exon 10 pattern was HT9SmS/1017HT (Table 2b). In the case of transfected cells, the primers INS1 and INS3 (Table 2b) were chosen to only amplify products arising from the constructs.

The RT-PCR experiments were done twice with the polyA⁺ RNAs and with total RNAs from three independent transfections, to ensure reproducibility. The RT-PCR product of SV10 whose length differed in size from that of spliced vector pSVIRB was cloned and sequenced. In Figures 1-4, the percent of the 10⁺ isoform was calculated by scanning the bands from three independent transfections and measuring the areas under the curves (by the IPLab Gel program from Scanalytics).

For the expression constructs, the predicted sizes of the 10⁻/10⁺ RT-PCR products are: 295/388 for SV10 and SV10L; 288/381 for SV9/10 and SV9/10L; 343/436 for SV10/11 and SV10/11L and 336/429 for SV9/10/11 and SV9/10L/11. Thus, the first four constructs give rise to products of almost identical length; ditto the last four.

### Blotting and hybridization

RT-PCR products from human polyA⁺ RNAs were run on a 6 % acrylamide/1x TBE gel. The gel was denatured in
1.5 M NaCl / 0.5 M NaOH for 30 min, renatured in
1.5 M NaCl / 0.5 M Tris (pH 7.2) for 30 min, electroblotted in the presence of lOx SSC on 0.20 mm Nytran (Schleicher and Schuell) and baked for 60 min at 80 °C.

The membrane was prehybridized for at least 3 h at 42 °C in a mixture of 50% deionized formamide, 5x SSC, 5x Denhardt's solution, 1% SDS, 100 mg/ml heat-denatured herring sperm DNA. To visualize the products, a probe was used which contained tau exon 9 and its proximal downstream intron (identical to the fragment used in the SV9 construct series).

The probe was labeled using the Amersham random priming kit and [³²P]-α-dCTP (Amersham PB.10205). Hybridization was carried out overnight (>17 hr) at 42 °C in the same solution to which the heat-denatured probe (∼5x10⁶ cpm/ml) was added. Following overnight hybridization, the membrane was washed (15 min at room temperature in 2x SSC / 0.1% SDS, 15 min at 52 °C in 0.1xSSC / 0.1% SDS, 15 min at 65 °C in 0.1xSSC / 0.1% SDS), wrapped in Saran wrap and exposed against Kodak X-Omat film at -70 °C in cassettes with intensifying screens. RT-PCR products from transfected constructs were run on 3% NuSieve agarose (FMC Bioproducts)/1x TAE gels.

### Results

Fig. 1B illustrates the RT-PCR determinded ratio between exon 10 inclusion (10⁺) and exon 10 exclusion/skipping (10⁻) of the minigene expression products, which are schematically shown in Fig. 1A and which have been obtained from Examples 1 and 3, in non-neuronal (COS) and neuroblastoma (SKN, NTs) cells. Primer pair INS1/INS3 was used for RT-PCR. It shows that the default pattern of exon 10 is inclusion, but its expression is modulated by the cellular environment.

Fig. 2B correspondingly illustrates the RT-PCR determinded ratio 10^{+/}10⁻ obtained in non-neuronal (COS; top panel) neuroblastoma (SKN; middle panel) and teratocarcinoma (NT2; bottom panel) cells, when using the minigene expression products shown in Fig. 2A, i.e. expression constructs which contain the short and long version of exon 10 flanked by various combinations of homologous and heterologous exons. The results shown in Fig. 2B indicate that the flanking exons of tau exon 10 are involved in its splicing regulation.

In addition, the RT-PCR results (using primer pair INS1/INS3) obtained in non-neuronal (COS) cells from the minigene constructs, which had been modified by mutations in the tau exon 10 sequence or its proximal downstream intron (cf. Fig. 3A), are shown in Fig. 3B. These results indicated that the splicing of exon 10 is affected by exonic elements and by its 5' splice site and proximal intron region.

Accordingly, it has been established that the minigene constructs according to the present invention are useful tools for the testing of substances which may change exon 10 splicing. They are particularly suitable as standard references for determining the effectiveness of substances to be capable of controlling tau exon 10 inclusion.

### Example 5

### Tau pre-mRNA processing analysis subsequent to transfection with tau exon 10 splicing regulators

RT-PCR was performed in a manner as described in Example 4 (using primer pair: INS1/INS3), except that mammalian cells were analyzed which had been 1:1 co-transfected with tau exon 10 containing minigene constructs (denoted SV 10) and with plasmids containing cDNA of various plicing regulators. The RT-PCR products are from 1:1 co-transfections of SV10 with the factors indicated in COS cells.

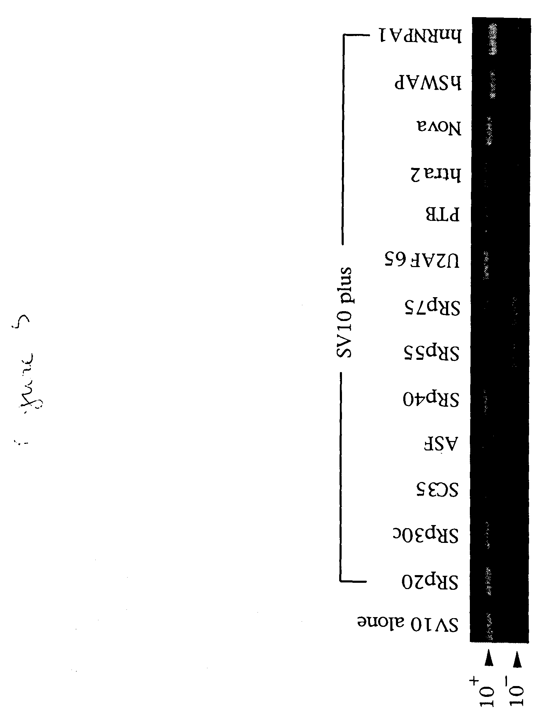

From the RT-PCR results shown in Fig. 4, it is observed that specific splicing regulators affect the expression of exon 10. It is establish that tau exon 10 inclusion is inhibited by the constitutive factors ASF/SF2, SRp55, SRp75, U2AF65 and the specific regulator hSWAP. Each of these shifts the ratio from the exclusive 10⁺ phenotype to varying degrees of inclusion of the 10⁻ isoform. These shifts are also seen in SV9/10L/11, although they are less dramatic due to the fact that this construct expresses a fair amount of 10⁻ isoform. On the other hand, the other tested splicing factors did not noticeably affect tau splicing by inhibiting exon 10 inclusion.

### Example 6

### Modification of tau exon 10 splicing by co-transfection with DNA encoding a cd2-like SR protein kinase

### Transfection

Transient transfection of adherent HEK293 cells was performed using the calcium phosphate method on 35 mm plates (six well tissue culture plate). The day before transfection 3.0 x10⁵ cells per plate were seeded in 3 ml DMEM/10% FCS. This lead to approximately 40-60% confluency on the day of transfection. After splitting, the cells were incubated at 37°C in 5% CO₂ for 20h.

Splicing assays were based on the titration of increasing amounts of plasmid DNA expressing a splicing factor to a constant concentration of minigene DNA. To avoid "squelching" effects, the 'empty' parental expression plasmid containing the promotor was added to ensure a constant amount of transfected DNA.

For the clk2 reaction, 2 µg of minigene DNA and and increasing amount of plasmid DNA expressing clk2 were used. This titration was from 0, 1, 2, 2.5 and 3 µg EGFP-clk2. The appropriate amount of empty vector (3, 2, 1, 0.5 and 0 µg) was added to ensure that equal amounts of DNA were transfected. The DNA solutions were brought to a total volume of 75 µl with water and 25 µl 1 M CaCl₂ were added. While mixing the DNA/CaCl₂ solution with a vortex, 100 µl of 2 x HBS were added dropwise.

The mixture was incubated for 10-20 min at room temperature to allow the calcium phosphate-DNA precipitate to form. The precipitates were resuspended by pipetting and the complete mixture was added dropwise to the cultured cells. The dishes were incubated at 37°C in 3% CO₂ overnight.

### RT-PCR analysis

RNA was isolated 18 hours after transfection using an RNeasy mini kit (Qiagen, Hilden, Germany) following the manufacturer's instructions. RNA was eluted in 40ml RNAse free H₂O. Best results were achieved when reverse transcription and following PCR are performed immediately after the RNA purification, thus avoiding freezing of the RNA or reverse transcription reaction. For reverse transcription, 2ml of isolated RNA were mixed with 5 pmol antisense specific primer in 0.5 µl H₂O, 2 ml 5xRT buffer, 1 µl 100 mM DTT, 1 µl 10 mM dNTP, 3 µl H₂O, 0.25 ml RNase inhibitor and 0.25 ml H⁻ reverse transcriptase. In one sample the RNA was substituted with water as a control. After a brief centrifugation, the tubes were incubated for 45 min in a 42°C water bath. The antisense specific primer used for tau minigene was: CAC CTC CAG TGC CAA GGT CTG AAG GTC ACC.
During this incubation period, the PCR mixture was prepared. It consisted of 50 pmol of sense and antisense primer each, 100 µl 10x PCR buffer, 20 µl 10 mM dNTPs in a total of 1000 µl water. The optimal MgCl₂ concentration for amplification was 1.5 mM final. Sense primer for the tau minigene was: CAG CTA CAG TCG GAA ACC ATC AGC AAG CAG.
For six reactions, 1 µl Taq polymerase was added to 300 µl PCR mixture. 2 ml of the RT reaction were added to 25 µl of this mix and PCR was performed.

The PCR program in a perkin elmer cycler was: Initial denaturation for 2 minutes at 94°C; 30 cycles: 20 seconds denaturation at 94°C, annealing at 60°C for 20 seconds, extension at 72°C for fourty seconds, after 30 cycles a final extension at 72°C for 7 min and cooling to 4°C.

Finally, the PCR reaction products were analyzed on a 0.3-0.4 cm thick 2% agarose TBE gel.

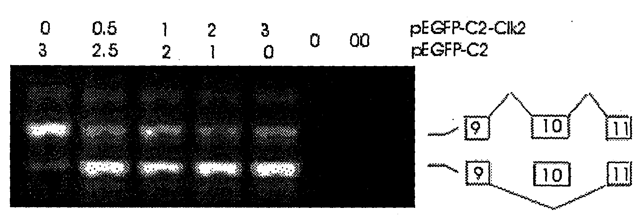

The results of the RT-PCR analysis after DNA transfection is shown in Fig. 5. It is clearly established that the cd2 like SR protein kinase clk2 inhibits exon 10 inclusion in a dose dependent manner. With increasing amounts of transfected DNA, the splicing pattern is shifted towards predominent tau exon 10 skipping (exclusion).

Therefore, the results obtained in Examples 5 and 6 establish that a "wrong" tau splicing pattern can be reversed in vivo. On this basis, the disease causing changes, i.e. the imbalance between tau exon 10 inclusion and exclusion, can be therapeutically treated by substances which are capable of controlling the alternative splicing pattern of the tau gene.

The present inventions relates to substances which are capable of controlling the inclusion of exon 10 of the tau gene. The substances can reverse "wrong" tau splicing pattern and. Since "wrong" tau splicing patterns are associated with tauopathies that cause dementia, the substances can be used as therapeutic agents. The invention also provides a method for testing substances that control tau exon 10 inclusion. The method is suitable for a high throughput screening. Additional products which are useful for changing tau exon 10 expression or for performing the test method are described as well.

## Claims

1. A substance which is capable of controlling the inclusion of exon 10 of the tau gene for use as a therapeutic agent.

2. The substance according to claim 1 which is a splicing regulator for the splicing of exon 10 of the tau gene.

3. The substance according to any one of claims 1 or 2 which is capable of at least partially suppressing the inclusion of exon 10 of the tau gene.

4. The substance according to claim 3 which is a polypeptide selected from the group consisting of U2AF65, hSWAP and the SR proteins ASF, SRp55, SRp75, or fragments, derivatives, or variants of these polypeptides being effective for suppressing the inclusion of exon 10 of the tau gene.

5. The substance according to claim 3 which is a DNA selected from the group consisting of
(i) DNAs which respectively encode for any one of the following polypeptides: U2AF65, hSWAP and the SR proteins ASF, SRp55 and SRp75, or
(ii) fragments, derivatives, alleles or mutant forms of the DNAs defined in (i), wherein said fragments, derivatives, alleles or mutant forms are effective for suppressing the inclusion of exon 10 of the tau gene.

6. The substance according to claim 1 which is a polypeptide controlling the phosphorylation of a splicing regulator for the splicing of the tau gene exon 10, or a DNA encoding such a polypeptide.

7. The substance according to claim 6, wherein the substance for controlling the phosphorylation of a splicing regulator is a SR protein kinase, or a DNA encoding said SR protein kinase.

8. The substance according to claim 7, wherein said substance is the cd2 like SR protein kinase, or a DNA encoding said cd2 like SR protein kinase.

9. The substance according to claim 6, wherein the substance for controlling the phosphorylation of a splicing regulator is a SR protein phosphatase, or a DNA encoding said SR protein phosphatase.

10. The substance according to claim 9 which is capable of at least partially stimulating the inclusion of exon 10 of the tau gene.

11. The substance being a DNA as defined in any one of claims 5 to 9, wherein the respective DNA is used for transfection of mammalian cells for being used as a therapeutic agent.

12. The substance according to claim 11, wherein the DNA is inserted into a vector which is suitable for transfecting mammalian cells.

13. The substance according to claim 12, wherein the inserted DNA is operatively linked to a expression control DNA sequence.

14. The substance according to any one of claims 1 to 13 which is used for the treatment of tauopathies.

15. The substance according to claim 14, wherein the tauopathy to be treated is frontotemporal dementia, Parkinsonism and/or Alzheimer's disease.

16. The substance according to claim 14, wherein the tauopathy to be treated is Pick's disease.

17. A process for changing the pre-mRNA processing relating to the tau gene of a mammalian cell, which comprises exposing the cell with a substance which is capable of controlling the inclusion of exon 10 of the tau gene.

18. The process according to claim 17, wherein the mammalian cell is transfected with a DNA selected from the following group of DNAs:
(a) a DNA encoding a polypeptide which is capable of at least partially suppressing the inclusion of exon 10 of the tau gene, and
(b) a DNA encoding a polypeptide which is capable of controlling the phosphorylation of a splicing regulator for exon 10 of the tau gene.

19. The process according to claim 18, wherein the DNA specified under (a) is selected from the group consising of:
(i) DNAs which respectively encode any one of the following polypeptides: U2AF65, hSWAP and the SR proteins ASF, SRp55 and SRp75, or
(ii) fragments, derivatives, alleles or mutant forms of DNAs defined in (i), wherein said fragments, derivatives, alleles or mutant forms are effective for suppressing the inclusion of exon 10 of the tau gene.

20. The process according to claim 18, wherein the DNA specified under (b) is a DNA encoding a SR protein kinase or a DNA encoding a SR protein phosphatase.

21. The process according to claim 20, wherein the DNA encodes for the cd2 like SR protein kinase.

22. The process according to any one of claims 18 to 21, wherein the DNA is inserted into a vector which is suitable for transfecting the mammalian cell.

23. A method for testing substances that control the inclusion of exon 10 of the tau gene, comprising the steps of:
(1) transfecting mammalian cells with a DNA which comprises exon 10 of the tau gene or fragments or mutant forms thereof,
(2) exposing said transfected cells from (1) to the substance to be tested, and
(3) determining the ratio between exon 10 inclusion and exon 10 skipping as a result of exposure step (2).

24. The method according to claim 23, wherein the DNA being used for transfection in step (1) is a minigene construct as defined in any one of claims 29 to 31.

25. The method according to claim 23, wherein step (3) includes a step of subjecting the RNA obtained from the transfected and exposed cells to reverse transcription, a step of amplifying the DNA obtained from reverse transcription such that exon 10 sequences of the tau gene may be amplified if present, and a step of performing a DNA analysis distinguishing between exon 10 inclusion and exon 10 skipping of the tau gene.

26. The method according to claim 25, wherein the DNA analysis is conducted by at least two probes, one probe being specific for exon 10 inclusion and one one probe being specific for exon 10 skipping.

27. The method according to claim 26, wherein the probes are molecular beacon probes.

28. The method according to claim 26 or 27, wherein the probe specific for exon 10 inclusion comprises an oligonucleotide sequence shown in SEQ ID NO. 2 or its complementary sequence, and the probe specific for exon 10 skipping comprises an oligonucleotide sequence shown in SEQ ID NO. 3 or its complementary sequence.

29. A minigene construct comprising the following DNA elements derived from the tau gene:
• exon 10 or fragments or mutant forms thereof,
• each flanking intron sequence upstream and downstream of exon 10, respectively, or fragments or mutant forms thereof, and
• exon 9 and/or exon 11, or fragments or mutant forms thereof.

30. A minigene construct according to claim 29, comprising a further foreign DNA element differing from the tau gene and serving as an internal splicing control.

31. The minigene construct according to claim 29, wherein said DNA elements are fused into a plasmid or a vector.

32. A mammalian host cell which is stably transfected with a DNA as defined in any one of claims 18 to 22.

33. A mammalian host cell which is stably transfected with a minigene construct as defined in any one of claims 29 to 31.
